(19)

Europäisches
Patentamt
European
Patent Office
Office européen
des brevets

(11) **EP 4 745 895 A1**

(12) **EUROPEAN PATENT APPLICATION**

(43) Date of publication:
**20.05.2026 Bulletin 2026/21**

(21) Application number: **24212582.1**

(22) Date of filing: **13.11.2024**

(51) International Patent Classification (IPC):
**G06T 11/00** (2026.01)

(52) Cooperative Patent Classification (CPC):
**G06T 12/30;** G06T 2211/408; G06T 2211/416;
G06T 2211/424; G06T 2211/444

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AE Eindhoven (NL)**

(72) Inventor: **BOERNERT, Peter Ulrich
Eindhoven (NL)**

(74) Representative: **Philips Intellectual Property &
Standards
High Tech Campus 52
5656 AG Eindhoven (NL)**

(54) **MEDICAL IMAGING WITH SPARSE CONSTRAINTS**

(57)    The present invention provides method and apparatus for medical imaging by applying image segmentation to an estimation of a medical image to obtain an anatomical and/or functional sparse constraint and reconstructing the medical image with respect to the anatomical and/or functional sparse constraint. This invention considers the anatomical and/or function sparsity inherent to medical images and allows conceptually to represent the medical image in an even more compact manner in the image domain, reducing the need of coherent samplings or information for the reconstruction. In this way, it is possible to reduce the number of sampled data and simplify the image reconstruction process, thereby increasing the efficiency and speed of medical imaging while ensuring the quality of the reconstructed image.

Fig. 1

**EP 4 745 895 A1**

**Description**

FIELD OF THE INVENTION

The invention relates to the field of medical imaging, and in particular to methods and apparatus for medical imaging with respect to sparse constraints.

BACKGROUND OF THE INVENTION

Medical imaging refers to the techniques and processes used to create visual representations of the interior of a body for clinical analysis and medical intervention. It plays a crucial role in diagnosing, monitoring, and treating diseases by allowing healthcare professionals to observe structures and functions inside the body without invasive surgery. The most common medical imaging modalities include X-rays, computed tomography (CT), magnetic resonance imaging (MRI), ultrasound, and nuclear medicine techniques such as positron emission tomography (PET).

Traditional medical imaging can be time-consuming due to several factors. First, the acquisition process, especially in modalities like MRI or CT, requires precise, time-consuming data collection to ensure high-quality images. Complex scans may involve multiple phases or angles, increasing the time needed. Additionally, processing and reconstructing these images from raw data into detailed visuals can be computationally demanding. Thus, there is a long need to accelerate the medical imaging process.

From a historical perspective, early modern medical imaging modalities, particularly MRI, as well as CT, PET, and others, were characterized by a segmented workflow. The processes of (a) raw data acquisition, (b) image reconstruction, (c) image analysis and post-processing, and (d) diagnosis were treated as distinct, independent stages. However, in recent advancements, the boundaries between these stages have progressively blurred. Data acquisition is increasingly integrated with image reconstruction, and reconstruction techniques are now benefiting from features traditionally applied during post-processing. This convergence reflects a more cohesive approach, enhancing the efficiency and quality of medical imaging practices as an integrated process.

Conventionally, advancements in accelerating image acquisition are primarily achieved through strategic under-sampling, guided by the principles of compressed sensing, combined with artificial intelligence, particularly for denoising applications. For example, in MRI, parallel imaging represents a crucial method to address the challenges of underdetermination, leveraging information from multiple receivers to simultaneously sample and observe data.

In the example of compressed sensing MRI (CS-MRI), the method exploits the inherent sparsity of medical images in specific transform domains, such as the wavelet, Fourier, or total variation domains, and reconstructs medical images using under-sampled random samples from the k-space (frequency domain). In the sparse domain, most image coefficients are zero or near-zero, allowing the image to be represented in a very compact form with fewer coefficients. The medical image is then reconstructed from the under-sampled k-space data by applying image sparsity constraints through an enhancement process. Typically, the reconstruction is enhanced by minimizing an objective function that includes a data consistency term and a regularization term enforcing image sparsity. These approaches enable image reconstruction with reduced data, thereby facilitating faster scanning processes.

However, there is an ongoing demand for further acceleration of data acquisition and medical image reconstruction, driven by the need to enhance efficiency while maintaining the medical image quality for diagnostic accuracy.

SUMMARY OF THE INVENTION

It is an object of the invention to provide solutions for efficient and accurate medical imaging which reconstructed medical images from under-sampled raw data, while ensuring the quality of the reconstructed medical image for diagnostic accuracy.

According to the invention, this object is addressed by the subject matter of the independent claims. Embodiments of the invention are described in the dependent claims.

Therefore, according to the invention, a medical imaging method is provided. The method proposes applying image segmentation to an estimation of a medical image to obtain an anatomical and/or functional sparse constraint and reconstruct the medical image with respect to the anatomical and/or functional sparse constraint.

Here, the term "image segmentation" refers to a process in which the medical image is partitioned into distinct regions or structures based on anatomical and/or functional characteristics. These distinct regions and structures may include organs, tissues, lesions, and other relevant features. At the pixel or voxel level, image segmentation refers to the process of clustering and segmenting pixels or voxels that belong to different distinct regions or structures.

The method can be computer-implemented. Specifically, the method can be implemented directly by a medical imaging device, such as MRT, CT or PET scanner. The medical image may be 2D images, 3D images and/or data with higher dimensions additionally including time, contrast evolution, and other features.

Theoretically, anatomical and/or physiological structures that share common properties at the cellular and functional level can be represented with the same or similar pixel/voxel values in a medical image, thus providing potential anatomical and/or functional sparsity. Image segmentation can cluster pixels/voxels that represent these linked anatomical and/or physiological structures and impose corresponding anatomical and/or functional sparse constraints for medical image reconstruction. Specifically, the anatomical sparse constraint may link anatomical and/or physiological structures that share common properties at the cellular level, while the functional sparse constraint may link structures that share common properties at the functional level.

The anatomical and/or functional sparse constraints conceptually allow the medical image to be represented in an even more compact manner in the image domain, reducing the need for coherent sampling or information for reconstruction. By reconstructing the medical image with respect to the anatomical and/or functional sparse constraints, it is possible to reduce the number of sampled data points and simplify the reconstruction process, thereby increasing the efficiency and speed of medical imaging while ensuring the quality of the reconstructed image.

This method can be combined with conventional compressed sensing theory. In addition to the data consistency term and image sparsity, the anatomical and/or functional sparsity is considered a term in the objective function to be minimized. In one embodiment of the present invention, the method may reconstruct the medical image by minimizing the objective function, which includes the data consistency term, the conventional regularization term, and the anatomical and/or functional sparse constraint. The conventional regularization term enforces image sparsity, while the anatomical and/or functional sparse constraint enforces anatomical and/or functional sparsity. The reconstruction may be executed through suitable iterative or enhancement methods, such as gradient descent, the alternating direction method of multipliers (ADMM), the iterative shrinkage-thresholding algorithm (ISTA), among others.

In an embodiment of the present invention, the medical image may be reconstructed through an iterative process where the steps of image segmentation and reconstruction are repeated. In the first iteration, the method applies image segmentation to an initial estimate of the medical image to obtain the anatomical and/or functional sparse constraint for reconstruction. In subsequent iterations, image segmentation is applied to the medical image reconstructed in the previous step to update the anatomical and/or functional sparse constraint. The method may receive the initial estimate of the medical image directly or generate the initial estimate based on acquired raw data.

Through the iterative process, the anatomical and/or functional sparse constraint adapts dynamically, further improving the accuracy of the reconstructed images.

Optionally, before applying image segmentation, the method may acquire raw data for reconstructing the medical image and generate an initial estimate of the medical image based on the raw data. The initial estimate of the medical image may be generated through direct back-projection or other non-iterative reconstruction methods. Compared to conventional medical imaging methods, the raw data can be under-sampled, as the anatomical and/or functional sparsity allows for reconstructing medical images with reduced data. The sampling points may be selected in a random or non-uniform manner.

This method allows reconstructing medical images with even fewer sampling points, thereby accelerating the data acquisition process and further increasing the efficiency and speed of medical imaging.

According to an embodiment of the present invention, when reconstructing the medical image through the iterative process, the method may terminate the iteration when certain termination criteria are met and output the image reconstructed in the latest iteration as the final medical image. The termination criteria may be configured according to specific needs. The method may terminate the iteration when the data consistency term is smaller than a predefined threshold, when a predefined number of iterations is reached, and/or when the objective function is smaller than a predefined threshold, indicating a global maximum.

According to an embodiment of the present invention, the method may refine the segmentation before reconstructing the medical image in terms of spatial scale and content. This is because within certain living structures, such as organs, which appear as tissue of similar kind and common properties, malfunctioning lesions could be present. Specifically, once the segmentation result is available, the method may analyze all pixels or voxels with appropriate statistical means, which might include several probabilistic tests (Z-score test, Interquartile Range (IQR) test, Grubbs' test, or machine learning based approaches DBSCAN (Density-Based Spatial Clustering of Applications with Noise) or Isolation Forests tests, all those could be of help to detect anomalies by considering the pixel/voxel data's distribution and density) to evaluate whether there are outliers and check whether these outliers form small local sub-structures which could be lesions, tumors, or inflammatory spots.

Based on the statistical results, the method may further refine the anatomical and/or functional sparse constraints for reconstructing the medical image. The refinement of the image segmentation helps avoid the mis-clustering of areas of interest, such as lesions, tumors, and inflammatory spots, thereby ensuring greater accuracy in the medical image reconstruction.

As discussed above, image segmentation may cluster the pixels or voxels belonging to different organs or tissues, such as white matter, grey matter, or cerebrospinal fluid, and impose the anatomical and/or functional sparse constraints. These sparse constraints may be expressed as constraints on the corresponding pixel or voxel values. The anatomical sparse constraint applies to the pixel/voxel values of anatomical and/or physiological structures that share common properties at the cellular level, while the functional sparse constraint applies to the structures that share common properties at the functional level. For example, these constraints can limit the average values for these pixels and voxels, where the average value may be tissue-specific and derived from signal simulations or from the statistics of the segmented and trusted pixels and voxels.

Optionally, the image segmentation may include organ-based segmentation, tissue-based segmentation, segmentation of anatomical sub-structures, and/or segmentation of functional sub-structures. In this approach, the pixels or voxels may be segmented and clustered with respect to different resolutions, imposing different anatomical and/or functional sparsity constraints for the medical imaging process. The type of image segmentation can be configured based on the needs or the part of the body under study.

The medical imaging method can be applied to different types of medical images, such as MRI, CT images, ultrasound images, and more. According to one embodiment of the present invention, the method is applied to reconstruct multiple MRIs of a single anatomy, where corresponding MR sequences can measure differently contrasted MR data. In this embodiment, the method may apply contrast-based image segmentations to the corresponding MRI images to obtain the anatomical and/or functional sparse constraints and then reconstruct the multiple images jointly based on the anatomical and/or functional sparse constraints.

In this embodiment, tailored and contrast-based segmentations identify voxels of different tissue types from individual MR contrasts, facilitating joint reconstruction and increasing the segmentation confidence and convergence speed. This approach can also be applied to spectral CT images, where tailored, spectrum-based segmentations are applied to the CT images at different energy levels.

According to another embodiment of the present invention, the method may be applied to reconstruct CT images. As discussed above, segmentation algorithms can differentiate not only between tissue types but also between organs and further anatomical/functional sub-structures. Thus, in each iteration, it is possible to apply different image segmentations to obtain different anatomical and/or functional sparse constraints and reconstruct the medical image based on these constraints. For example, in each iteration, constraints may be imposed for different organs within the region under study. In this way, the confidence of the segmentation is further increased, improving the quality of the reconstructed medical image.

A second aspect of the invention provides a medical imaging apparatus that reconstructs the medical image according to the method discussed above. The apparatus comprises an image segmentation module configured to apply image segmentation to an estimate of a medical image to obtain an anatomical and/or functional sparse constraint and a reconstruction module configured to reconstruct the medical image with respect to the anatomical and/or functional sparse constraint.

The apparatus can be a device capable of generating medical images. For example, it can be an MRI scanner, CT scanner, PET scanner, or other medical imaging devices used for real-time medical imaging. The apparatus can also be computers or servers capable of communicating with the scanners to receive raw image data for post-imaging processing. These computers and servers can be centralized or distributed.

Optionally, the image segmentation module and the reconstruction module are configured to perform the steps of image segmentation and reconstruction iteratively. In the first iteration, the estimation of the medical image is an initial estimate, and in subsequent iterations, the estimation is based on the medical image reconstructed in the previous iteration. The reconstruction module may be configured to reconstruct the image through an enhancement process aimed at minimizing an objective function. The objective function comprises the data consistency term, the conventional regularization term, and the anatomical and/or functional sparse constraint, as discussed above.

According to one embodiment of the present invention, the apparatus may further comprise a data acquisition module configured to acquire raw data for reconstructing the medical image and an initialization module configured to generate an initial estimate of the medical image based on the raw data. The data acquisition module may be the medical imaging scanner or simply a module that receives raw data sampled by the scanner.

In another embodiment, the apparatus further comprises an output module configured to terminate the iteration once the termination criteria discussed above are met and to output a final medical image. The apparatus may output the medical image to a display or a printer.

A third aspect of the present invention provides a computer program product comprising instructions that, when executed by a computer, cause the medical imaging apparatus to generate medical images based on the method discussed above.

A fourth aspect of the present invention provides a computer-readable medium that stores the computer program product.

BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:

Fig. 1 schematically depicts a flow chart of a medical imaging method according to an embodiment of the invention,
Fig. 2 schematically depicts a flow chart of the medical imaging method according to another embodiment of the invention,
Fig. 3 schematically depicts a flow chart of the medical imaging method according to another embodiment of the invention, and
Fig. 4 schematically depicts a block diagram of a medical imaging apparatus according to an embodiment of the invention.

DETAILED DESCRIPTION OF EMBODIMENTS

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

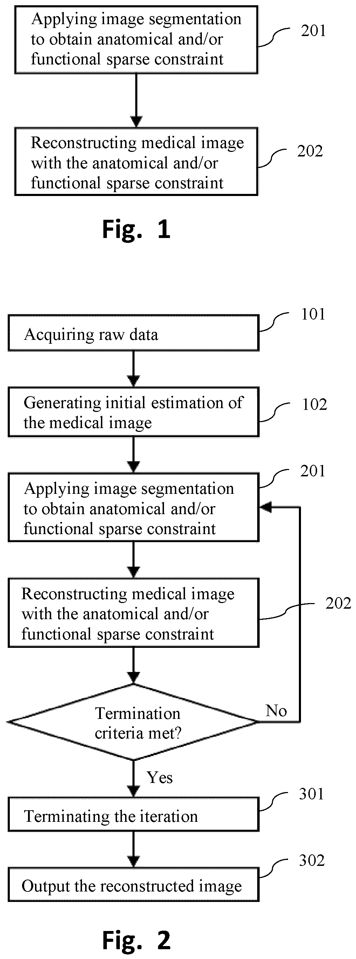

Fig. 1 depicts a flow chart of a medical imaging method according to an embodiment of the invention. The method comprises the following steps:

201: applying image segmentation to an estimation of a medical image to obtain an anatomical and/or functional sparse constraint, and
202: reconstructing the medical image with respect to the anatomical and/or functional sparse constraint.

The method can be computer-implemented and executed by a device capable of generating medical images. For example, the method can be executed directly by an MRI scanner, CT scanner, PET scanner, or other medical imaging devices. The method can also be executed by a computer or server capable of receiving raw image data. The computers and servers can be centralized or distributed.

In one embodiment of the present invention, the anatomical and/or functional sparse constraint is taken into account in addition to conventional compressed sensing theory. Thus, the imaging device performs the enhancement of the medical image by:
reconstructing the medical image x by minimizing an objective function comprising a data consistency term, a conventional regularization term and the anatomical and/or functional sparse constraints.

The objective function may be expressed as equation (1):

$$\{x\} = \underset{x}{\mathrm{argmin}}\|Ax - d\|_2^2 + \lambda_1\|H(x)\|_* + \lambda_2\|T(x)\|_* \qquad (1)$$

In equation (1), $\|Ax - d\|$ is the data consistency term ensuring that the reconstructed image matches the actual acquired data; $\lambda_1\|H(x)\|_*$ is the conventional regularization term, enforcing the image sparsity $H(x)$ of the reconstructed image, as used in compressed sensing. The conventional regularization term can be understood as image sparse constraints of the reconstruction process. $\lambda_2\|T(x)\|_*$ represents the anatomical and/or functional sparse constraints obtained via image segmentation which enforce anatomical and/or functional sparsity $T(x)$ of the reconstructed image. In one implementation, the anatomical and/or functional sparsity $T(x)$ can be a function that thresholds pixel or voxel values outside a certain disallowed range, replacing them with an average tissue value (with the option of adding a small amount of noise, as noise is not always detrimental). The range values are tissue-specific, as is the average, and can be derived from signal simulations or from the statistics of segmented and trusted voxels. The regularization parameters $\lambda_1$ and $\lambda_2$ can be determined empirically using several test cases and visual result inspection. It is also possible to engage small artificial intelligence (AI) models to learn and enhance the regularization parameter, based on the data input, noise level and other signal or data characteristics.

The dimension of the equation depends on the dimension of the image x. The medical image x may be 2D images, 3D images and/or images with higher dimensions like time, contrast evolution, and other features.

In one specific embodiment, the device reconstructs the medical imaging using an iterative process as depicted in Fig. 2. At the beginning of the iteration, the device applies image segmentation to an initial estimation of the medical image to obtain the anatomical and/or functional sparse constraint and reconstructs intermediate estimation of the medical image. Then the device applies image segmentation to this intermediate estimation to update the anatomical and/or functional sparse constraint for the next reconstruction step.

As shown in Fig. 2, the initial estimation of the medical image may be generated by the imaging device. Thus, the method further comprises the steps:

101: acquiring raw data for the to be reconstructed medical image, and
102: generating the initial estimation of the medical image based on the raw data.

In specific examples, the device may acquire the raw data from a storage device or a database to reconstruct the medical image. In some specific examples, the device may acquire the image data in real-time via an MR scanner, CT scanner, ultrasonic scanner, and similar devices. The initial estimation of the medical image can be generated using simple and conventional techniques, such as direct back projection. The initial estimation can also be generated using conventional compressed sensing techniques.

Fig. 2 also depicts the termination of the iteration. The device may be configured to terminate the iteration when the quality of the medical image is sufficiently high. This termination criterion may be quantified by various measures, such as when the data consistency term is smaller than a certain threshold, when the value of the objective function is below a certain threshold, and/or when the number of iterations reaches a predefined limit, e.g., predetermined number of iterations. Other termination criteria may also be used, for example, it is possible to engage AI models to evaluate the quality of the reconstructed medical image. Once it is determined that the quality of the reconstructed medical image is sufficiently high, the iterative process is terminated. In some embodiments the threshold may be selected by a user through a user interface.

Thus, in response to determining a termination criterion is met, the method may perform the following steps:

301: terminating the iteration, and
302: outputting a final medical image.

The final medical image is the medical image reconstructed in the last iteration and may be output to a display or printer for assisting diagnosis.

According to this embodiment, a medical imaging process may include the following: first, raw data for reconstructing the medical image may be under-sampled by a medical image scanner, which could be an MRI scanner, CT scanner, or similar device; then, an initial estimation of the medical image may be generated based on the under-sampled raw data, and image segmentation may be applied to the initial estimation to obtain an anatomical and/or functional sparse constraint. As the next step, an intermediate estimation of the medical image may be reconstructed with respect to the anatomical and/or functional sparse constraint and fed back to the image segmentation step to update the anatomical and/or functional sparse constraint. A new intermediate estimation of the medical image is then reconstructed based on the updated constraint and fed back to the image segmentation step again. Specifically, the steps of image segmentation and reconstruction may be repeated until the termination criterion is met, ensuring that the quality of the medical image reconstructed from the under-sampled raw data is sufficient. Image segmentation may cluster and segment structures of similar types of tissue. However, within certain living structures, such as organs, which exhibit tissue with common properties, malfunctioning lesions, tumors, and inflammatory spots may be present. These sub-structures are of interest in medical imaging and should be identified. Thus, the method may propose: refining the image segmentation before reconstructing the medical image to identify these sub-structures.

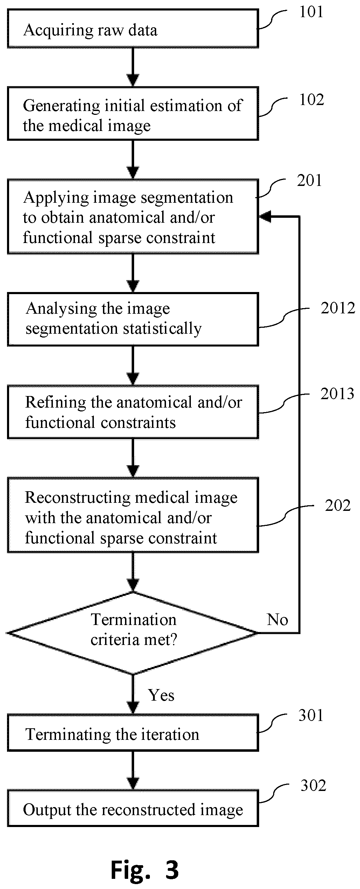

Fig. 3 depicts schematically the method refining the image segmentation. The refinement was implemented by the steps:

2012: analyzing the result of the image segmentation statistically to determine local sub-structure, and
2013: refining the anatomical and/or function sparse constraints in response to determining local sub-structure.

During the refinement, all pixels and voxels of the intermediate medical images are analyzed using corresponding statistical tools to identify outliers, which may indicate the presence of lesions, tumors, or inflammatory spots. These spots should then be distinguished from normal tissues and trigger the refined anatomical and/or functional models to impose additional sparse constraints.

These sparse constraints may be expressed as constraints on the corresponding pixel or voxel values. The anatomical sparse constraint comprises constraints on pixel/voxel values of anatomical and/or physiological structures which share common properties on cellular level, and the functional sparse constraint comprises constraints on pixel/voxel values of anatomical and/or physiological structures which share common properties on functional level. For example, in MRI, all pixels belonging to white matter may be clustered and constraint to have values within appropriate thresholds. Virtually, in an extreme case, one could assume all pixel have kind of "same" pixel value. In this term, all these pixels could be regarded as one virtual pixel to add sparsity for image reconstruction.

Based on types of the medical images and the structures to be studied, the image segmentation can be organ-based segmentation, tissue-based segmentation, segmentation of anatomical sub-structures and/or segmentation of function sub-structures. For example, for T2 weighted MRI contrast, modern segmentation algorithm may cluster the pixels either to white matter, to grey matter or to cerebrospinal fluid. It can work for other contracts as well, as for example, for flow imaging, where constraints could be put on the expected flow velocity boundaries, or diffusion MRI, where constraints could be put on the ADC (apparent diffusion coefficient) value. For CT images, segmentation may be organ-based and impose different constraints on the pixel or voxel value for different organs.

In one specific example, the method may be applied to multiple MRI contrasts, including T1-weighted contrast, T2-weigheted contrast and other contracts. In this example, corresponding MR sequences capture differently contrasted MR data, and the method may:

applying contrast-based image segmentations to the multiple MRI images of different image contrasts to obtain the anatomical and/or functional sparse constraint, and
reconstructing jointly the multiple MRI images based on the anatomical and/or functional sparse constraint.

This embodiment employs contrast-based image segmentations on the respective MRI contrasts to obtain anatomical and/or functional sparse constraints and reconstructs the multiple contrasts jointly based on these constraints.

In another specific example, organ-based segmentation is applied to CT image. In this embodiment, different organs may be identified in each iteration via different image segmentation algorithms. The method may propose:

in different iterations, applying different image segmentations to obtain adaptive anatomical and/or functional sparse constraints, and
reconstructing the CT image based on the adaptive anatomical and/or functional sparse constraints.

For instance, in the initial iterations, only the pixels corresponding to the heart are clustered and enhanced, while in subsequent iterations, the pixels associated with the kidneys are clustered and processed, and so forth. This stepwise approach enables the identification of individual organs or tissues at each stage, allowing for the independent processing of each structure. By segmenting and enhancing specific organs or tissues separately in distinct iterations, the method reduces the burden on the segmentation algorithm while simultaneously ensuring the accuracy and precision of the segmentation. This incremental and focused clustering improves the reliability of organ identification and enhances the overall quality of the reconstructed medical images.

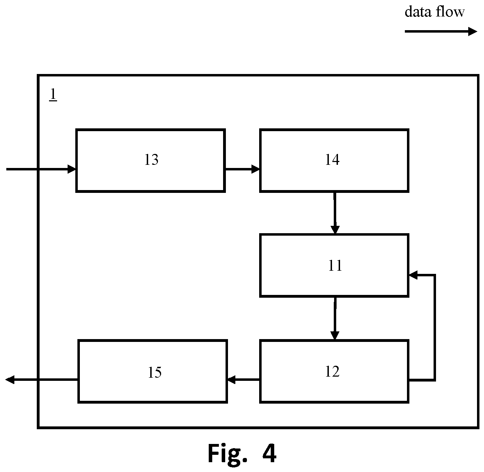

Fig. 4 depicts a block diagram of a medical imaging apparatus 1 according to the invention. The apparatus is configured to produce medical images according to the method as discussed above. Specifically, the apparatus comprises at least one image segmentation module 11 and a reconstruction module 12. The image segmentation module 11 is configured to apply image segmentation to the estimation of a medical image to obtain the anatomical and/or functional sparse constraint while the reconstruction module 12 is configured to reconstruct the medical image with respect to the anatomical and/or functional sparse constraint.

In addition, the image segmentation module 11 can be configured to refine the image segmentation by statistically analyzing the results of the image segmentation to identify lesions, tumors, and/or inflammatory spots for refining the sparse constraints. In specific applications, the image segmentation module 11 can also be configured to apply contrast-based image segmentation to respective MRI contrasts or apply different segmentation algorithms to CT images in different iterations. The image segmentation module 11 can also perform other image pre-processing steps, such as denoising.

Based on the segmentation results and the adapted anatomical and/or functional sparse constraints, the reconstruction module 12 may be configured to reconstruct the images accordingly. Specifically, when the segmentation and the sparse constraints are refined, the reconstruction module is configured to reconstruct the images based on the refined anatomical and/or functional sparse constraints. When tailored, contrast-based segmentations are applied to different MRI contrasts, the reconstruction module 12 may be configured to jointly reconstruct the multiple MRI contrasts based on the corresponding constraints. This approach also applies to CT images, where different image segmentations are applied in different iterations. The reconstruction module 12 may then be configured to reconstruct the medical image according to the different constraints in corresponding iterations.

Both the image segmentation module 11 and the reconstruction module 12 may be configured to enhance the medical image iteratively. As depicted in Fig. 4, intermediate medical images reconstructed in a previous iteration are fed back to the image segmentation module for iterative enhancement.

The apparatus can be applied to, or can be, a device capable of generating medical images. For example, it can be an MRI scanner, CT scanner, PET scanner, or other medical imaging devices used for real-time medical imaging. The apparatus can also be computers or servers capable of communicating with the scanners to receive raw image data for post-imaging processing. These computers and servers can be centralized or distributed. Additionally, the apparatus can be applied to devices for parallel MRI, spectral CT, or other advanced medical imaging techniques.

As shown in Fig. 4, the apparatus 1 may further comprise a data acquisition module 13 configured to acquire raw data for reconstructing the medical image, and an initialization module 14 configured to generate an initial estimation of the medical image based on the raw data. During operation, the data acquisition module 13 may receive or sense raw image data for image reconstruction. Due to the introduction of anatomical and/or functional sparsity, the raw data can be under-sampled. The raw data is then fed into the initialization module 14 to generate an initial estimation of the medical image through a simple process, such as direct back projection or other basic methods. The initial estimation is then input into the image segmentation module to begin the iterative or non-iterative reconstruction.

The apparatus 1 may further comprise an output module 15, which is configured to check the termination criteria, to terminate the iteration in response to determining a certain termination criterion is met and to output the medical image. In this regard, the final medical image may be output to an external display or a display integrated into the apparatus. The final medical image can also be output to corresponding medical image printers for assisting future diagnostic.

It is understood that one or more of the aforementioned embodiments of the invention may be combined as long as the combined embodiments are not mutually exclusive.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to our advantage. Any reference signs in the claims should not be construed as limiting the scope. Further, for the sake of clearness, not all elements in the drawings may have been supplied with reference signs.

**Claims**

1. A medical imaging method comprising:

   applying (201) image segmentation to an estimation of a medical image to obtain an anatomical and/or functional sparse constraint, and
   reconstructing (202) the medical image with respect to the anatomical and/or functional sparse constraint.

2. The medical imaging method according to claim 1, wherein reconstructing (202) the medical image comprises:
   reconstructing (2021) the medical image by minimizing an objective function comprising a data consistency term, a conventional regularization term and the anatomical and/or functional sparse constraint.

3. The medical imaging method according to claim 1 or 2, wherein the medical image is reconstructed by iteratively performing the steps of image segmentation (201) and reconstructing (202),

   in a first iteration, the estimation of the medical image is an initial estimation of the medical image, and
   in subsequent iterations, the estimation of the medical image is a medical image reconstructed in a previous iteration.

4. The medical imaging method according to any of claims 1 to 3, further comprising:

   before applying (201) image segmentation, acquiring (101) raw data for reconstructing the medical image, and
   generating (102) the initial estimation of the medical image based on the raw data.

5. The medical imaging method according to claim 3 or 4, further comprising:

   terminating (301) the iteration when a termination criterion is met, and
   outputting (302) a final medical image,

wherein the termination criterion comprises: a data consistency term is smaller than a threshold, a pre-determined number of iterations is reached, and/or the objective function is smaller than a predefined threshold.

6. The medical imaging method according to any of claims 1 to 5, further comprising:
refining the image segmentation before reconstructing the medical image.

7. The medical imaging method according to claim 6, wherein refining the image segmentation comprises:

analyzing (2012) a result of the image segmentation statistically to determine local sub-structure, and
refining (2013) the anatomical and/or function sparse constraints in response to determining local sub-structure.

8. The medical imaging method according to claim 7, wherein the local sub-structure comprises lesion, tumor and/or inflammatory spot.

9. The medical imaging method according to any of claims 1 to 8, wherein

the anatomical sparse constraint comprises constraints on pixel/voxel values of anatomical and/or physiological structures which share common properties on cellular level, and/or
the functional sparse constraint comprises constraints on pixel/voxel values of anatomical and/or physiological structures which share common properties on functional level.

10. The medical imaging method according any of claims 1 to 9, wherein the image segmentation comprises: organ-based segmentation, tissue-based segmentation, segmentation of anatomical sub-structures and/or segmentation of functional sub-structures.

11. The medical imaging method according to any of claims 1 to 10, wherein the medical image comprises multiple magnetic resonance images, MR, images of different image contrasts, the method comprising:

applying (2014) contrast-based image segmentations to the multiple MR images of different image contrasts to obtain the anatomical and/or functional sparse constraint, and
reconstructing (2024) jointly the multiple MR images based on the anatomical and/or functional sparse constraint.

12. The medical imaging method according to any of claims 1 to 10, wherein the medical image comprises computed tomography, CT, image, the method comprising:

in different iterations, applying (2015) different image segmentations to obtain adaptive anatomical and/or functional sparse constraints, and
reconstructing (2025) the CT image based on the adaptive anatomical and/or functional sparse constraints.

13. A medical imaging apparatus (1) for generating a medical image according to a method of any of claims 1 to 12, the device comprising:

an image segmentation module (11) configured to apply image segmentation to an estimation of a medical image to obtain an anatomical and/or functional sparse constraint, and
a reconstruction module (12) configured to reconstruct the medical image with respect to the anatomical and/or functional sparse constraint.

14. A computer program product comprising instructions which, when executed by a computer, causes an apparatus (1) of claim 13 to carry out the steps of a method according to any of the claims 1 to 12.

15. A computer-readable medium having stored thereon the computer program product of claim 14.

```
┌─────────────────────────────┐
│ Applying image segmentation │  ⌐ 201
│ to obtain anatomical and/or  │
│ functional sparse constraint │
└─────────────────────────────┘
              │
              ▼
┌─────────────────────────────┐
│ Reconstructing medical image │  ⌐ 202
│ with the anatomical and/or   │
│ functional sparse constraint │
└─────────────────────────────┘
```

**Fig. 1**

```
┌─────────────────────────────┐
│ Acquiring raw data           │  ⌐ 101
└─────────────────────────────┘
              │
              ▼
┌─────────────────────────────┐
│ Generating initial estimation of │  ⌐ 102
│ the medical image            │
└─────────────────────────────┘
              │
              ▼
┌─────────────────────────────┐
│ Applying image segmentation  │  ⌐ 201
│ to obtain anatomical and/or  │◄──┐
│ functional sparse constraint │   │
└─────────────────────────────┘   │
              │                    │
              ▼                    │
┌─────────────────────────────┐   │
│ Reconstructing medical image │⌐ 202
│ with the anatomical and/or   │   │
│ functional sparse constraint │   │
└─────────────────────────────┘   │
              │                    │
              ▼                    │
         ◇ Termination ◇  No       │
         ◇ criteria met? ◇ ────────┘
              │
              │ Yes
              ▼
┌─────────────────────────────┐
│ Terminating the iteration    │  ⌐ 301
└─────────────────────────────┘
              │
              ▼
┌─────────────────────────────┐
│ Output the reconstructed image │  ⌐ 302
└─────────────────────────────┘
```

**Fig. 2**

```
┌─────────────────────────────┐
│  Acquiring raw data         │──── 101
└─────────────────────────────┘
              │
              ▼
┌─────────────────────────────┐
│  Generating initial         │──── 102
│  estimation of              │
│  the medical image          │
└─────────────────────────────┘
              │
              ▼
┌─────────────────────────────┐
│  Applying image segmentation│──── 201
│  to obtain anatomical and/or│◄──┐
│  functional sparse constraint│   │
└─────────────────────────────┘   │
              │                    │
              ▼                    │
┌─────────────────────────────┐   │
│  Analysing the image        │──── 2012
│  segmentation statistically │   │
└─────────────────────────────┘   │
              │                    │
              ▼                    │
┌─────────────────────────────┐   │
│  Refining the anatomical    │──── 2013
│  and/or functional          │   │
│  constraints                │   │
└─────────────────────────────┘   │
              │                    │
              ▼                    │
┌─────────────────────────────┐   │
│  Reconstructing medical     │──── 202
│  image with the anatomical  │   │
│  and/or functional sparse   │   │
│  constraint                 │   │
└─────────────────────────────┘   │
              │                    │
              ▼              No    │
          ◇ Termination ◇─────────┘
          ◇ criteria met? ◇
              │
              │ Yes
              ▼
┌─────────────────────────────┐
│  Terminating the iteration  │──── 301
└─────────────────────────────┘
              │
              ▼
┌─────────────────────────────┐
│  Output the reconstructed   │──── 302
│  image                      │
└─────────────────────────────┘
```

# Fig. 3

data flow →

**Fig. 4**

Europäisches
Patentamt
European
Patent Office
Office européen
des brevets

# EUROPEAN SEARCH REPORT

**Application Number**

EP 24 21 2582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/011489 A1 (UNIV SYDNEY [AU]) 28 January 2016 (2016-01-28) * abstract * * paragraphs [0006] - [0018] * * figure 3 * | 1-5,9, 10,12-15 | INV. G06T11/00 |
| A | US 2020/241096 A1 (BUSTIN AURELIEN [GB] ET AL) 30 July 2020 (2020-07-30) * paragraphs [0001] - [0021] * * abstract * | 5 | |
| A | CN 117 456 029 A (UNIV EAST CHINA NORMAL) 26 January 2024 (2024-01-26) * the whole document * | 1-15 | |
| A | US 2011/058719 A1 (TRZASKO JOSHUA D [US] ET AL) 10 March 2011 (2011-03-10) * the whole document * | 1-15 | |

|  |
|---|
| **TECHNICAL FIELDS SEARCHED (IPC)** |
| G06T |

The present search report has been drawn up for all claims

| Place of search | Date of completion of the search | Examiner |
|---|---|---|
| Munich | 24 April 2025 | Klemencic, Ales |

EPO FORM 1503 03.82 (P04C01)

## ANNEX TO THE EUROPEAN SEARCH REPORT
## ON EUROPEAN PATENT APPLICATION NO.

EP 24 21 2582

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2025

| Patent document cited in search report | Publication date | Patent family member(s) | | Publication date |
|---|---|---|---|---|
| WO 2016011489 A1 | 28-01-2016 | EP | 3171782 A1 | 31-05-2017 |
| | | US | 2017172534 A1 | 22-06-2017 |
| | | WO | 2016011489 A1 | 28-01-2016 |
| US 2020241096 A1 | 30-07-2020 | GB | 2580695 A | 29-07-2020 |
| | | US | 2020241096 A1 | 30-07-2020 |
| CN 117456029 A | 26-01-2024 | NONE | | |
| US 2011058719 A1 | 10-03-2011 | NONE | | |

EPO FORM P0459

For more details about this annex : see Official Journal of the European Patent Office, No. 12/82